(19) Europäisches Patentamt
European Patent Office
Office européen des brevets

(11) EP 1 758 172 A1

(12) **EUROPEAN PATENT APPLICATION**
published in accordance with Art. 158(3) EPC

(43) Date of publication:
**28.02.2007 Bulletin 2007/09**

(21) Application number: **05750987.9**

(22) Date of filing: **06.06.2005**

(51) Int Cl.:
***H01L 29/786*** *(2006.01)* ***H01L 51/00*** *(2006.01)*
*C07D 333/10 (2006.01)*

(86) International application number:
**PCT/JP2005/010324**

(87) International publication number:
**WO 2005/122278 (22.12.2005 Gazette 2005/51)**

(84) Designated Contracting States:
**GB**

(30) Priority: **10.06.2004 JP 2004172317**
**04.04.2005 JP 2005107214**

(71) Applicant: **KONICA MINOLTA HOLDINGS, INC.**
**Tokyo 100-0005 (JP)**

(72) Inventors:
- **TANAKA, Tatsuo**
**c/o Konica Minolta Techn.Ctr. Inc.**
**Hachioji-shi,**
**Tokyo 1918511 (JP)**
- **HIRAI, Katsura**
**c/o Konica Minolta Techn.Ctr. Inc.**
**Hachioji-shi,**
**Tokyo 1918511 (JP)**
- **TAKEMURA, Chiyoko**
**c/o Konica Min. Techn.Ctr. Inc.**
**Hachioji-shi,**
**Tokyo 1928505 (JP)**
- **KATAKURA, Rie**
**c/o Konica Minolta Techn. Ctr. Inc.**
**Hachioji-shi,**
**Tokyo 1928505 (JP)**
- **KITA, Hiroshi**
**c/o Konica Minolta Techn. Ctr. Inc.**
**Hachioji-shi,**
**Tokyo 1928505 (JP)**

(74) Representative: **Rees, Alexander Ellison et al**
**Urquhart-Dykes & Lord**
**30 Welbeck Street**
**GB-London W1G 8ER (GB)**

# (54) ORGANIC SEMICONDUCTOR THIN FILM, ORGANIC SEMICONDUCTOR DEVICE, ORGANIC THIN FILM TRANSISTOR, AND ORGANIC ELECTRO-LUMINESCENCE ELEMENT

(57) An organic semiconductor thin film, comprising an organic semiconductor compound, wherein the organic semiconductor thin film is manufactured by a process of forming a film by using a solution or a dispersion at room temperature prepared by mixing the organic semiconductor compound and an organic solvent, and the half width of a diffraction peak having the maximum intensity is 0.4° or less in an X-ray diffraction spectrum of the film.

FIG. 1 (a)

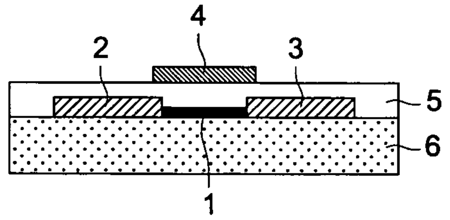




Printed by Jouve, 75001 PARIS (FR)

(Cont. next page)

FIG. 1 ( b )

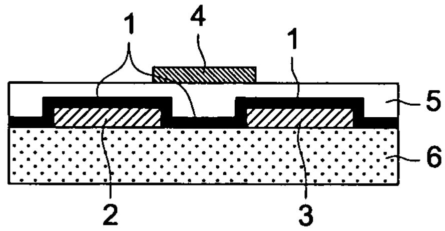


FIG. 1 ( c )

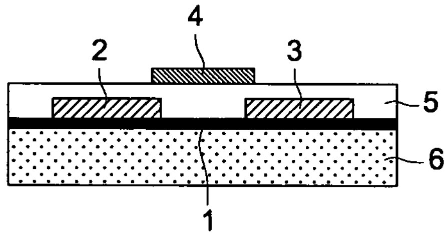


FIG. 1 ( d )

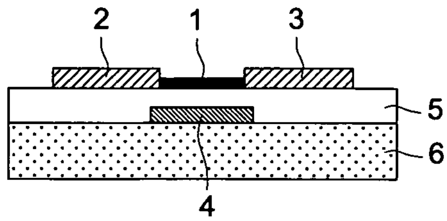


FIG. 1 ( e )

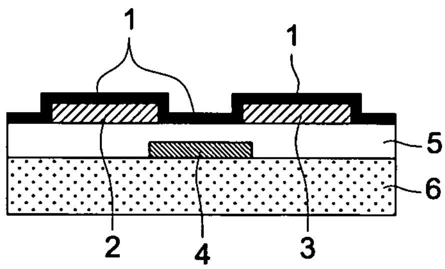


FIG. 1 ( f )

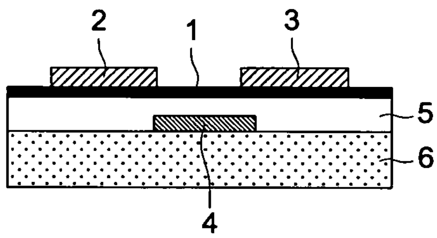

## Description

### FIELD OF THE INVENTION

The invention relates to an organic semiconductor thin film, an organic semiconductor device, an organic thin film transistor and an organic electronic luminescence element.

### BACKGROUND ART

Need of flat panel display for computer rises accompanied with spreading of information terminals. Moreover, electronic paper or digital paper as a thin, light and easily mobile displaying medium is needed because the chance of providing information in a form of electronic signals instead of paper medium is increased accompanied with the progress of information system.

In the planar displaying apparatus, the displaying medium is generally constituted by the use of an element applying liquid crystal, electronic luminescence element (hereafter, referred as organic EL) or electrophoresis. In such the displaying medium, technology in which an active driving element (TFT element) is principally applied for obtaining a uniformity of the brightness and a high rewrite speed of the image. In usual displays for computer, for example, the TFT elements are formed on a glass substrate and the liquid crystals or the organic EL elements are sealed.

Here, in the production of the TFT element, the production process including a vacuum process using a vacuum chamber has to be repeatedly applied for forming the constituting layers. Consequently, the costs for equipment and running the production become very high. For the TFT element, processes such as a vacuum deposition, doping and photolithography should be repeatedly performed for each of the layers. Therefore, the element is formed on the substrate through several tens processes. In a semiconductor portion making the important point of switching action, plural kinds of layer such as a p-type semiconductor layer and an n-type semiconductor layer are laminated. In such the processes for producing the silicon semiconductor, the change of the equipment corresponding to the requirement of large-sizing of the displaying image is difficult because a largely changing in the design of the production apparatus such as the vacuum chamber is necessary.

However, in the production of the TFT element, the production process including a vacuum process using a vacuum chamber has to be repeatedly applied for forming the constituting layers. Consequently, the costs for equipment and running the production become very high. For the TFT element, processes such as a vacuum deposition, doping and photolithography should be repeatedly performed for each of the layers. Therefore, the element is formed on the substrate through several tens processes. In a semiconductor portion making the important point of switching action, plural kinds of layer such as a p-type semiconductor layer and an n-type semiconductor layer are laminated. In such the processes for producing the silicon semiconductor, the change of the equipment corresponding to the requirement of large-sizing of the displaying image is difficult because a largely changing in the design of the production apparatus such as the vacuum chamber is necessary.

Further, the material of the substrate is limited to one having a resistivity against heating in the processes since the usual production processes for the TFT using silicon include a process performed at high temperature. Consequently, glass is only practically usable. Therefore, the displaying apparatus becomes one which is heavy, lacking in the flexibility and easily broken by falling when the displaying apparatus is constituted by the usual TFT elements. Such the properties caused by forming the TFT elements on the glass substrate are not suitable for satisfying the requirements for the light mobile thin display accompanied with the progress of the information system.

Besides, studying on organic semiconductor material having high charge transfer ability is aggressively progressed.

It may be possible to liquefy an organic semiconductor material by suitably improving its molecular structure so that an organic semiconductor layer can be formed by a printing method including an inkjet method and coating by making the obtained organic semiconductor liquid to an ink.

Though it may impossible to produce the semiconductor element by such the low temperature process by using the usual silicon type semiconductor material, it may be possible with respect to the device employing the organic semiconductor. Therefore, the limitation on the heat resistivity of the substrate is alleviated and, for example, the TFT element may be possible to be formed on a transparent resin substrate plate. When the TET elements can be formed on the transparent resin substrate plate and the displaying materials can be drove by the TFT elements, the display will be made to one lighter in the weight, higher in the flexibility than those of the usual one and is hardly or difficultly broken by falling.

The organic semiconductor materials investigated so far for realizing TFT devices are conjugate polymeric compounds such as, polyphenylene vinylene, polypyrrole, polythiophene, etc., (see, for example, Non-Patent Documents 1 to 3) or their oligomers (see, for example, Patent Document 2), polyacene compounds such as anthracene, tetracene, pentacene, etc. (see, for example, Patent Document 1).

Thiophene polymers typified by P3HT are soluble in organic solvents and can be used for manufacturing using a low temperature process such as the above. However, when an organic semiconductor layer is formed using a material having a molecular weight distribution such as a polymer, amorphous parts with random arrangement are formed in considerable numbers within the layer. In such amorphous parts, the overlapping of π-conjugated surface of the thiophene ring is small, and since the carrier movement speed is controlled, satisfactory TFT characteristics have not been obtained.

On the other hand, polyacene compounds typified by pentacene have high crystallinity because of strong cohesive force between the molecules, and because of this, it has been reported that high carrier mobility and superior semiconductor device characteristics are manifested. In addition, it has been reported that high carrier mobility is manifested by using an evaporated film in which pentacene is arranged in a highly ordered manner (see, for example, Non-Patent Document 4).

From these reports, in order to obtain an organic semiconductor thin film that manifests superior TFT characteristics, it is considered very important that a crystalline structure in which the molecules are arranged in a highly regular manner within the organic semiconductor film is present. However, since these polyacene compounds either do not dissolve or are difficult to dissolve in organic solvents, there was the problem that the manufacturing could not be done by coating.

Further, although even thiophene oligomers having no substituent groups, typified by unsubstituted sexythiophene, can easily form π-stacks between molecules and can form a structure with orderly arrangement, they are insoluble like pentacene, and there was the problem that it was not possible to manufacture films except only by evaporation. (See Patent Document 2.)

In the above manner, it was difficult to obtain a film with high crystallinity and orderly arrangement of molecules while being soluble in an organic solvent.

In order to solve the problems such as the above, pentacene to which solubility has been given by introducing alkyl chains has been proposed (see, for example, Non-Patent Document 5). However, a high temperature is necessary for dissolving in an organic solvent said alkyl substituted pentacene, and also, the solubility was not sufficient. In addition, although aromatic halogenized hydrocarbons such as trichlorobenzene, etc., have been used to dissolve said alkyl substituted pentacene, but non-halogenic solvents are desirable than these halogen based solvents from the point of view of suitability with the environment, and there were also the problems in manufacturing such as problems in solubility, etc.

Further, α,ω-alkyl thiophene oligomers with alkyl chains introduced at the ends of the oligomers have been proposed (see, for example, Non-Patent Document 6). These thiophene oligomers can be dissolved in an organic solvent such as chloroform, etc., and it is possible to form films by coating. However, even in the case of these materials, operations such as heating, etc., are necessary for dissolving in an organic solvent, and sufficient solubility has not been obtained.

Patent Document 1: Japanese Unexamined Patent Application Publication No. Hei 5-55568.
Patent Document 2: Japanese Unexamined Patent Application Publication No. Hei 8-264805.
Non-Patent Document 1: "Science" Magazine, No. 289, p. 599 Non-Patent Document 2: "Nature" Magazine, No. 403, p. 521 Non-Patent Document 3: Advanced Material magazine, No. 2 of the year 2002, p. 99
Non-Patent Document 4: Appl. Phys. Lett., 1998, 72, 1854
Non-Patent Document 5: Proc. ICSM-2004
Non-Patent Document 6: Chemical Material magazine, 1998, No. 10, p. 633

## DISCLOSURE OF THE INVENTION

The purpose of the present invention is to solve the above problems, and to provide an organic EL device provided with an organic semiconductor device having a high carrier mobility, an organic thin film transistor, and said device or said transistor, using an organic semiconductor thin film that can be manufactured by coating.

The above purpose of the present invention has been achieved by the following structures 1 to 18.

Item 1

An organic semiconductor thin film with the feature that, in an organic semiconductor thin film that includes an organic semiconductor compound, it is manufactured by mixing said organic semiconductor compound and an organic solvent, and produced by passing through a process of forming the film using a solution of a dispersed liquid at room temperature, and also, the half width of the diffraction peak of the maximum intensity is 0.4° or less in the X-ray diffraction spectrum of said film.

Item 2

An organic semiconductor thin film with the feature that, in an organic semiconductor thin film that includes an organic semiconductor compound, it is manufactured by mixing said organic semiconductor compound and an organic solvent, and produced by passing through a process of forming the film using a solution of a dispersed liquid at room temperature, and also, the half width of the diffraction peak of the maximum intensity is 0.2° or less in the X-ray diffraction spectrum of said film.

Item 3

An organic semiconductor thin film according to Item 1 or Item 2 with the feature that said organic solvent includes a non-halogen based solvent.

Item 4

An organic semiconductor thin film according to any one of Items 1 to 3 with the feature that the weight average molecular weight Mw of said organic semiconductor compound is 10000 or less.

Item 5

An organic semiconductor thin film according to any one of Items 1 to 4 with the feature that, the ratio (Mw/Mn) of the weight average molecular weight Mw to the numeric average molecular weight Mn of said organic semiconductor compound is 2 or less.

Item 6

An organic semiconductor thin film according to any one of Items 1 to 5 with the feature that, the content of said organic semiconductor compound in said film is 95% or more.

Item 7

An organic semiconductor thin film of any one of Items 1 to 6 with the feature that, said organic semiconductor compound is a $\pi$-conjugated compound that includes 2 or more aromatic group rings.

Item 8

An organic semiconductor thin film according to Item 7 with the feature that said organic semiconductor compound has as its partial structure at least two or more types of aromatic hydrocarbon rings or at least two or more types of aromatic heterocyclic rings.

Item 9

An organic semiconductor thin film according to Item 7 with the feature that said organic semiconductor compound has as its partial structure at least three or more types of aromatic hydrocarbon rings or at least three or more types of aromatic heterocyclic rings.

Item 10

An organic semiconductor thin film according to any one of Items 7 to 9 with the feature that said organic semiconductor compound has as its partial structure unsubstituted aromatic hydrocarbon rings that do not have condensed rings, or has as its partial structure unsubstituted aromatic heterocyclic rings.

Item 11

An organic semiconductor thin film according to any one of Items 1 to 10 with the feature that said organic semiconductor compound includes thiophene oligomer that has as its partial structure thiophene rings with a substituent group, and at least 2 or more unsubstituted thiophene ring repetitions are in succession.

Item 12

An organic semiconductor thin film according to Item 11 with the feature that the number of thiophene rings included in said thiophene oligomer is 3 to 20.

Item 13

An organic semiconductor thin film according to Item 11 with the feature that the number of thiophene rings included in said thiophene oligomer is 4 to 10.

Item 14

An organic semiconductor thin film according to any one of Items 11 to 13 with the feature that said thiophene oligomer has a partial structure expressed by the following General Equation (1).

Chemical Equation 1:

General Equation (1)

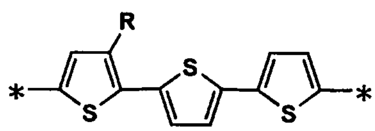

[R in this equation represents a substituent group.]

Item 15

An organic semiconductor thin film according to any one of Items 11 to 14 with the feature that the end groups of said thiophene oligomer do not have a thienyl group.

Item 16

An organic semiconductor thin film according to any one of Items 11 to 15 with the feature that, no Head-to-Head structure is present within the structure of said thiophene oligomer.

Item 17

An organic semiconductor device with the feature that, said organic semiconductor device is provided with an organic semiconductor thin film according to any one of Items 1 to 16.

Item 18

An organic thin film transistor with the feature that, an organic semiconductor thin film according to any one of Items 1 to 16 has been used as the organic semiconductor layer.

Item 19

An organic electroluminescence device with the feature that it is provided with an organic semiconductor device according to Item 17 or an organic thin film transistor according to Item 18.

Using the organic semiconductor thin film according to the present invention, it was possible to provide an organic TFT with a high carrier mobility, a field effect transistor, and in addition, a switching device having said organic TFT or said field effect transistor.

## BRIEF DESCRIPTION OF THE DRAWINGS

Figure 1 is a diagram showing a sample configuration of an organic TFT according to the present invention.
Figure 2 is an example of an outline equivalent circuit of the organic TFT according to the present invention.
Figure 3 is an example of the X-ray diffraction spectrum of the organic semiconductor thin film according to the present invention.
Figure 4 is an example of the X-ray diffraction spectrum of the organic semiconductor thin film according to the present invention.
Figure 5 is an example of the X-ray diffraction spectrum of the organic semiconductor thin film according to the present invention.
Figure 6 is an example of the X-ray diffraction spectrum of the comparison organic semiconductor thin film.
Figure 7 is a schematic diagram showing an example of an organic EL device having a sealed structure.
Figure 8 is a schematic diagram showing an example of a substrate having a TFT used in the organic EL device.

## BEST MODE FOR CARRYING OUT THE INVENION

In the organic semiconductor thin film according to the present invention, by using a configuration stipulated by any one of Claims 1 to 16, it is possible to obtain an organic semiconductor thin film that is useful for application to thin film transistors. Further, it is known that the organic thin film transistors (known as organic TFTs) prepared using said organic semiconductor thin films exhibit high carrier mobility, and exhibit superior transistor characteristics such as good ON/OFF characteristics.
In pentacene etc., in which good TFT characteristics have been reported so far, it is known that molecules are arranged in an orderly manner while forming $\pi$-stacks between molecules. However, in polythiophene such as PHT, etc., organized arrangement between molecules is formed only partially. In other words, when a polythiophene compound described in the conventional widely known literature, etc., $\pi$-stacks are formed only in parts because it is a very big polymer molecule, and since the parts not related to $\pi$-stacks remain in large numbers as the parts with random arrangement, it is predicted that it is not possible to obtain sufficient carrier mobility or ON/OFF characteristics.
After the inventors of the present invention carried out various investigations of the above problems, in the case of materials having high crystallinity such as single crystals in which the molecules are arranged in an orderly manner, in the X-ray diffraction spectrum obtained by Cu-K$\alpha$ characteristics X-rays, it is known that diffraction peaks with a very small half widths are obtained. Therefore, taking the half widths of diffraction peaks as an index of the orderliness of the molecular arrangement within the film, it was found that materials forming films that exhibit smaller half width diffraction peaks form organic semiconductor thin films that manifest superior TFT characteristics.
In particular, as in thiophene oligomers related to the present invention, by carrying out molecular design of oligomers adjusted to have molecular numbers within a specific range (this has the same meaning as adjusting the number of repetition counts to be within a specific range) while providing a solubility site (thiophene ring site having a substituent group) and a $\pi$-stack forming site (sites of successive unsubstituted thiophene rings), and by using said oligomer, it is possible to form coated films having ideal molecular arrangements such as those seen in conventionally well-known pentacene etc., and as a result, the present inventors succeeded in greatly improving the TFT characteristics.
The details of the different structural elements related to the present invention are described below in sequence.

«Organic Semiconductor Film»

The organic semiconductor film related to the present invention is described here.

«Solution or Dispersed Liquid at Room Temperature»

The organic semiconductor thin film of the present invention is prepared by passing through a process of film formation using a solution or a dispersed liquid at room temperature prepared by mixing an organic semiconductor compound (organic semiconductor compounds are discussed later) and an organic solvent given below. Here, a solution or a dispersed liquid at room temperature means that a solution or a dispersed liquid is formed when the organic semiconductor compound and an organic solvent are mixed together under a temperature condition of 10 °C to 80 °C, and a dispersed liquid indicates that the organic semiconductor compound is dispersed in the liquid in the particle state, and includes the condition in which the organic semiconductor compound has dissolved partially within the dispersed liquid. Further, as one form of the dispersed liquid is, for example, the condition in which the compound dissolves at a temperature condition of 80 °C thereby forming a solution, but when returned to room temperature (normally this is a

temperature around 25 °C), the particles, coagulates, and precipitates of the organic semiconductor compound are dispersed in the organic solvent.

(Organic Solvent)

Although there is no particular restriction, the organic solvents related to the present invention can be single solvent or mixed solvents, and desirably, non-halogen based solvents are used. The non-halogen based solvents used in the present invention can be aliphatic solvents such as hexane, octane, etc., cycloaliphatic solvents such as cyclohexane, etc., aromatic solvents such as benzene, toluene, xylene, etc., ether type solvents such as tetrahydrofuran, dioxane, ethylene-glycol-diethyl-ether, anisole, benzyl-ethyl-ether, ethyl-phenyl-ether, diphenylether, methyl-t-butyl-ether, etc., ester type solvents such as methyl acetate, ethyl acetate, ethyl cellosolve, etc., alcoholic solvents such as methanol, ethanol, isopropanol, etc., ketone type solvents such as acetone, methyl-ethyl-ketone, cyclohexanone,2-hexanone, 2-heptanone, 3-heptanone, etc., or other solvents such as dimethyl-formamide, diethyl-sulfoxide, diethyl-formamide, 1,3-dioxolan, etc.

Further, the organic solvents that are used simultaneously, although not particularly restricted, can desirably be methanol, ethanol, isopropanol, acetone, methylethyl-ketone, methyl-iso-butyl-ketone, pyrrolidone, N-methylpyrrolidone, dimethyl-formamide, dimethyl-acetoamide, methyl acetate, ethyl acetate, butyl acetate, methyl lactate, ethyl lactate, butyl lactate, β-methyl-methoxy-propionate, β-ethyl-ethoxy-propionate, propylene glycol monomethyl ether acetate, toluene, xylene, hexane, limonene, cyclo-hexane, etc. It is also possible to use combinations of two or more types of these solvents.

Further, as ester type solvents, it is possible to use alkyl-ester-oxy-isobutyrate, etc., and as ester-oxy-isobutyrates, it is possible to use α-alkoxy-isobutyrate alkyl esters such as methyl-α-methoxy-isobutyrate, ethyl-α-methoxy-isobutyrate, methyl-α-ethoxy-isobutyrate, ethyl-α-ethoxy-isobutyrate, etc., β-alkoxy-isobutyrate alkyl esters such as methyl-β-methoxy-isobutyrate, ethyl-β-methoxy-isobutyrate, methyl-β-ethoxy-isobutyrate, ethyl-β-ethoxy-isobutyrate, etc., and α-hydroxy-isobutyrate alkyl esters such as methyl-α-hydroxy-isobutyrate, ethyl-α-hydroxy-isobutyrate, and, in particular, it is possible to use methyl-α-methoxy-isobutyrate, methyl-β-methoxy-isobutyrate, methyl-β-ethoxy-isobutyrate, or methyl-α-hydroxy-isobutyrate.

«X-ray Diffraction Spectrum of Organic Semiconductor Compounds»

After the organic semiconductor compounds related to the present invention are mixed with the above organic solvents and a film is formed using the prepared solution, in the X-ray diffraction spectrum of the obtained film, the feature is that the half width of the diffraction peak with the maximum intensity is less than or equal to 0.4°, desirably 0.3° or less, and more desirably 0.2° or less.

In general, it is possible to guess the extent of crystallinity of compounds from the half width of the diffraction peak in the X-ray diffraction spectrum, and a diffraction peak with a very small half width is obtained from a material in which the molecules are arranged in an orderly manner over a wide region.

In the present invention, by using an organic semiconductor thin film that is effectively soluble in an organic solvent at room temperature, and also yields a diffraction peak with a small half width in the X-ray diffraction spectrum, we have succeeded in obtaining an organic TFT device that manifests desirable semiconductor device characteristics such as high carrier mobility, etc.

While the measurement of the X-ray diffraction spectrum related to the present invention is made using the apparatus and measurement conditions given below, the substrate (base) used at the time of measuring the X-ray diffraction spectrum of the organic thin film of the present invention can be the same as or can be different from the substrate (base) used in the organic thin film transistor (organic TFT) of the present invention, and the data with the smaller value of the half width of the maximum intensity obtained from the measured X-ray diffraction spectrum is used as the "half width of the diffraction peak of the maximum intensity in the X-ray diffraction spectrum of the film" of the present invention.

Here, an example is given below of the measurement conditions of measuring the X-ray diffraction spectrum using the X-ray diffraction apparatus RINT-TTR2 (manufactured by Rigaku Denki). In addition, depending on the sample, in some cases, even if the measurement conditions are changed, it is confirmed that the same values are obtained for the diffraction peak and the half width yielding the maximum intensity. The film thickness of the organic films used during the measurements is in the range of 5 nm to 100 nm, and should desirably be in the range 10 nm to 50 nm.
(Measurement Conditions)
X-ray tube: Cu (Cu-Kα characteristics X-rays are used)
Voltage: 50.0 kV
Current: 300.0 mA
Start angle: 2θ = 2.00 deg.

Stop angle: 2θ = 45.00 deg
Step angle: 0.020 deg/step
Measurement time: 0.40 seconds/step

(Method of Calculating Half Width)

Taking the background on the low angle and wide angle side of the diffraction peak, the sum of the intensities of all the measurement points above that background is obtained, and this is taken as the area of the peak. Among the measurement points, the one with the highest intensity is taken as the height of the peak, and the half width is calculated according to the following equation.

$$\text{Half width} = \text{SF} \times \text{Area}/\text{Height}$$

SF: A constant related to the shape of the peak, and is set as 0.85 in the present invention.
The above operations were done using JADE6 (manufactured by Materials Data. Inc.).
Further, the film used in the X-ray diffraction measurement can be obtained by covering on a base such as a substrate the solution having the organic semiconductor, which solution is the organic semiconductor compound dissolved in an organic solvent, and evaporating said organic solvent by a method such as heating, etc. The method of covering the solvent having the organic semiconductor compound can be coating, spraying, or contacting the base directly with the solution, etc., and in concrete terms, the methods of casting, spin coating, dip coating, screen printing, ink jet printing, blade coating, etc., are well known.
These operations can be done in air, or in an inert gas environment such as Argon gas, etc. In addition, at the time of evaporating the solvent, it is also possible to control the temperature of the base, the pressure, temperature, etc., of the environment. Further, it is also possible to contact the base with the solution containing organic semiconductor compound in the supersaturated state, thereby forming a film of organic semiconductor on the surface of the base. The organic semiconductor film formed on the base using these methods, can further be subjected to heating or cooling, applying electric field, magnetic field, or temperature gradient, etc., or application of pressure, friction or other processings, thereby improving the orientation within the film.
Although there is no restriction on the film thickness of the organic semiconductor film that is formed, it is desirable that the film thickness is 100 nm or less, and more desirably, 50 nm or less.
Further, although there is no restriction on the base used, it can be a silicon substrate, a glass substrate, a polymer film, etc. Also, the surface of the base that becomes the boundary between the base and the organic semiconductor film can also be processed by a well-known method such as forming a thermal oxidation film, etc., or the surface can be modified by processing using alkyl trichlorosilane, etc.

«Molecular Weight and Molecular Weight Distribution (Mw, Mn) of Organic Semiconductor Compound»

It is desirable that the molecular weight of the organic semiconductor compound of the present invention (the weight average molecular weight) is 10000 or less, and still more desirably in the range of 100 to 5000. In addition, it is desirable that the ratio of the weight average molecular weight (Mw) to the numeric average molecular weight (Mn) of the organic semiconductor compound of the present invention (the molecular weight distribution) is 2 or less.
The measurement of the weight average molecular weight (Mw) and the numeric average molecular weight (Mn) of the organic semiconductor compound of the present invention was made by carrying out molecular weight measurement using GPC (Gel Permeation Chromatography) using THF (tetra-hydro-furan) as the column solvent.
In specific terms, for 1 mg of the measurement sample, 1 ml of THF (use the material after air in it has been removed) is added, and is dissolved thoroughly by stirring using a magnetic stirrer at room temperature. Next, after processing with a membrane filter with a pore size of 0.45 μm to 0.50 μm, it is injected into the GPC apparatus.
The GPC measurement conditions are, stabilizing the column at 40 °C, flowing THF at a flow rate of 1 ml per minute, and measuring while injecting about 100 μl of the sample with a density of 1 mg/ml. As a column, it is desirable to use a combination of commonly available polystyrene gel columns. For example, a combination of Shodex GPC KF-801, 802, 803, 804, 805, 806, and 807 manufactured by Showa Denko, or a combination of TSKgelG1000H, G2000H, G3000H, G4000H, G5000H, G6000H, G7000H, and TSK guard column manufactured by Toso can be used.
A refractive index detector (RI Detector) or a UV detector is used desirably as the detector. In the measurement of molecular weight of the sample, the molecular weight distribution of the sample is calculated using a detected weight curve prepared using single dispersion polystyrene standard particles. It is desirable to use about 10 points as the polystyrene for preparing the detected weight curve.

In the present invention, the molecular weight measurement was made under the following measurement conditions.

(Measurement Conditions)

Apparatus: HLC-8020 (Manufactured by Toso)
Columns: GMHXLx2, G2000HXLx1
Detector: RI and/or UV
Solution flow rate: 1.0 ml/min.
Sample density: 0.01 g/20 ml
Quantity of sample: 100 μl
Weight detection curve: Prepared using standard polystyrene

«Content of Organic Semiconductor Compound»

Further, it is desirable that the quantity of organic semiconductor compound in the organic semiconductor film is 95% by mass or more, still more desirably 98% by mass or more. Also, when not having effect on the organic thin film transistor of the present invention, the concrete compound of the organic semiconductor compound of the present invention, although described later, can be the same material or can be a mixture of several different compounds with different structures.

Here, the content of the organic semiconductor compound was analyzed using HPLC (High Speed Liquid Chromatography). The measuring apparatus and the measurement conditions are given below.
HPLC Apparatus: GULLIVER manufactured by Nihon Bunko
Column: Wako Pack Wakosil-II 5SIL-100 (manufactured by Wako Pure Chemicals)
Eluting solution: Toluene (special grade)/cyclohexane (special grade) mixed solution
Column temperature: 40 °C
Flow rate: 1 ml/min
Detector: UV/VIS (310 nm)
Injection quantity: 400 μl

«Compounds Desirable to be Included as Organic Semiconductor Compound»

As the organic semiconductor compound of the present invention, it is suitable to use π-conjugated compounds, and compounds having the following features are used desirably.

(a) Said organic semiconductor compound is π-conjugated compound that includes two or more aromatic rings. Here, an aromatic ring is any of aromatic hydrocarbon ring, aromatic heterocyclic ring, and aromatic condensed ring. Also, the aromatic rings include can be the same or can be different.
(b) Said π-conjugated compound has two or more types of aromatic hydrocarbon rings or two or more types of aromatic heterocyclic rings as a partial structure.
(c) Said π-conjugated compound has three or more types of aromatic hydrocarbon rings or three or more types of aromatic heterocyclic rings as a partial structure.
(d) The π-conjugated compound described in (a), (b), or (c) above has unsubstituted aromatic hydrocarbon ring without a condensed ring, or unsubstituted aromatic heterocyclic ring as a partial structure.

(π-conjugated compound)

As the π-conjugated compound of the present invention, it is possible to use a conventionally well-known semiconductor material if it satisfies the conditions as the above organic semiconductor material (solubility in an organic solvent at room temperature, half width of 0.4° or less of the maximum intensity in the X-ray diffraction spectrum of the formed film).

For example, acene types such as pentacene and tetracene, pthalocyanine types including lead pthalocyanine, low molecular compounds such as perylene or its tetracarboxylic acid derivative, hexametric thiophene called α-thienyl or sexythiophene, aromatic oligomers such as fluorene oligomer, etc., and in addition, conjugated polymers such as polythiophene, polythienylenevinylene, poly-p-phenylenevinylene, etc., can be used.

From the point of view of the effects described in the present invention (obtaining organic semiconductor film by coating, and also, obtaining an organic TFT with a high carrier mobility using said organic semiconductor film), it is desirable that the π-conjugated compound of the present invention is a π-conjugated compound that includes two or

more aromatic rings, and further, it is desirable to use a π-conjugated compound that satisfies the conditions described in (b) or (c) above.

As the organic semiconductor compound of the present invention, it is desirable that the π-conjugated compound has two or more types of aromatic hydrocarbon rings or two or more types of aromatic heterocyclic rings as a partial structure.

Here, as the aromatic hydrocarbon rings, it is possible to use benzene ring, biphenyl ring, naphathalene ring, azulene ring, anthracene ring, phenanthrene ring, pyrene ring, chrysene ring, naphtacene ring, triphenylene ring, o-terphenyl ring, m-terphenyl ring, p-terphenyl ring, acenaphthene ring, coronene ring, fluorene ring, fluoranthrene ring, naphthacene ring, pentacene ring, perylene ring, pentaphene ring, picene ring, pyrene ring, pyranthrene ring, anthranthrene ring, etc. In addition, it is also possible to use substituent group of the thiophene oligomer to be described later.

Further, as the aromatic heterocyclic rings, it is possible to use furan ring, thiophene ring, oxazole ring, pyridine ring, pyridazine ring, pyrimidine ring, pyrazine ring, triazine ring, benzoimidazole ring, oxadiazole ring, triazole ring, imidazole ring, pyrazole ring, thiazole ring, indole ring, benzoimidazole ring, benzothiazole ring, benzooxadiazole ring, quinoxaline ring, quinazoline ring, phthalazine ring, carbazole ring, carboline ring, diazacarbazole ring (this is a carboline ring in which one more carbon atom in its constituent hydrocarbon ring is replaced by a nitrogen atom). In addition, said aromatic heterocyclic ring can also have a substituent group in a thiophene oligomer to be described later.

While most of the conventionally well-known π-conjugated compounds require the use of a vacuum deposition process for forming an organic semiconductor film, the organic semiconductor compound of the present invention make it possible to prepare thin film transistors by a film forming method that is not available in the conventionally well-known organic semiconductors because these films can be placed on various types of substrates (these can be the substrate for forming the organic thin film transistor, or can be another substrate) using an atmospheric pressure process such as coating or printing.

Further, in the case of the conventionally well-known polymers or some of the oligomers, although a substituent group has been introduced in their molecular structure in order to improve the solubility in the solvent thereby making it possible to form thin films using those solvents, since regular arrangement among molecules is formed only partially, it could not be said to be sufficient in terms of charge mobility and durability, and in particular, in the thiophene oligomer of the present invention, by providing solubility sites (thiophene ring sites having substituent groups) and π-stack formation sites (sites with successive unsubstituted thiophene rings), and making the oligomer one with its molecular weight adjusted to be in a specific range (this is the same as adjusting the number of repetitions to be within a specific range), the coated film is formed to have the same ideal molecular arrangement as seen in conventionally well-known pentacene, etc., and success has been achieved in greatly improving the organic TFT characteristics.

A compound most preferably usable as an organic semiconductor compound relevant to the present invention is explained.

«Thiophene oligomer»

Thiophene oligomer relevant to the present invention is explained.

The thiophene oligomer relating to the invention has a thiophene ring having a substituent and a partial structure which includes a repetition unit of an unsubstituted thiophene ring continues at least two or more, and the number of the thiophene rings contained in the thiophene oligomer is preferably from 3 to 40, more preferably from 3 to 20, and still more preferably from 4 to 10. Further more preferably, the thiophene oligomer has a partial structure represented by General formula (1).

<<Thiophene oligomer represented by Formula 1>>

Thiophene oligomers represented by Formula 1 preferably employed in the invention are described below.

Examples of the substituent represented by R in Formula 1 include an alkyl group such as a methyl group, an ethyl group, a propyl group, an isopropyl group, a tert-butyl group, a pentyl group, a hexyl group, an octyl group, a dodecyl group, a tridecyl group, a tetradecyl group and a pentadecyl group; a cycloalkyl group such as a cyclopentyl group, a cyclohexyl group; an alkenyl group such as a vinyl group and an allyl group; an alkynyl group such as an ethynyl group and a propalgyl group; an aryl group such as a phenyl group, a p-chlorophenyl group, a mesityl group, a tolyl group, a xylyl group, a naphthyl group, an anthoryl group, an azurenyl group, an acenaphthenyl group, a fluorenyl group, a phenathoryl group, an indenyl group, a pyrenyl group and a biphenyl group; an aromatic heterocyclic group such as a furyl group, a thienyl group, a pyridyl group, a pyridazyl group, pyrimidyl group, a triazyl group, an imidazolyl group, a pyrazolyl group, a thiazolyl group, a benzimidazolyl group, a benzoxazolyl group, a quinazolyl group and a phtharadyl group; a heterocyclic group such as a pyrrolidyl group, an imidazolidyl group, a morpholyl group and a oxazolidyl group; an alkoxyl group such as a methoxy group, an ethoxy group, a propyloxy group, a pentyloxy group, a hexyloxy group and an octyloxy group; a cycloalkoxy group such as a cyclopentyloxy group and a cyclohexyloxy group; an aryloxy group

such as a phenoxy group and a naphthyloxy group; an alkylthio group such as a methylthio group, an ethylthio group, a propylthio group, a pentylthio group, a hexylthio group, an octylthio group and a dodecylthio group; a cycloaalkylthio group such as a cyclopentylthio group and a cyclohexylthio group; an arylthio group such as a phenylthio group and a nephthylthio group; an alkoxycarbonyl group such as a methyloxycarbonyl group, an ethyloxycarbonyl group, a butyloxylcarbonyl group, an octyloxycarbonyl group and a dodecyloxycarbonyl group; an aryloxycarbonyl group such as a phenyloxycarbonyl group and a naphthyloxycarbonyl group; a sulfamoyl group such as an aminosulfonyl group, a methylaminosulfonyl group, a dimethylaminosulfonyl group, a butylaminosulfonyl group, a hexylaminosulfonyl group, a cyclohexylaminosulfonyl group, an octylaminosulfonyl group, a dodecylaminosulfonyl group, a phenylaminosulfonyl group, a naphthylaminosulfonyl group and a 2-pyridylaminosulfonyl group; an acyl group such as an acetyl group, an ethylcarbonyl group, a propylcarbonyl group, a pentylcarbonyl group, a cyclohexylcarbonyl group, a naphthylcarbonyl group and a pyridylcarbonyl group; an acyloxy group such as an acetyloxy group, an ethylcarbonyloxy group, a butylcarbonyloxy group, an octylcarbonyloxy group, a docecylcarbonyloxy group and a phenylcarbonyloxy group; an amido group such as a methylcarbonylamino group, an ethylcarbonylamino group, a dimethylcarbonylamino group, a propylcarbonylamino group, a pentylcarbonylamino group, a cyclohexylcarbonylamino group, a 2-ethylhexylcarbonylamino group, an octylcarbonylamino group, a dodecylcarbonylamino group, a phenylcarbonylamino group and a naphthylacarbonylamino group; a carbamoyl group such as an aminocarbonyl group, a methylaminocarbonyl group, a dimethylaminocarbonyl group, a propylaminocarbonyl group, a pentylaminocarbonyl group, a cyclohexylaminocarbonyl group, an octylaminocarbonyl group, a 2-ethylhexylaminocarbonyl group, a dodectlaminoocarbonyl group, a phenylaminocarbonyl group, a naphthylaminocarbonyl group and a 2-pyridylamino-carbonyl group; a ureido group such as a methylureido group, an ethylureido group, a pentylureido group, a cyclohexylureido group, an octylureido group, a dodecylureido group, a phenylureido group, a naphthylureido group and a 2-pyridylureido group; a sulfinyl group such as a methylsulfinyl group, an ethylsulfinyl group, a butylsulfinyl group, a cyclohexylsulfinyl group, a 2-ethylhexylsulfinyl group, a dodecylsulfinyl group and a phenylsulfinyl group; an alkylsulfonyl group such as a methylsulfonyl group, an ethylsulfonyl group, a butylsulfonyl group, a cyclohexylsulfonyl group, a 2-ethylhexylsulfonyl group and a dodecylsulfonyl group; an arylsulfonyl group such as a phenylsulfonyl group, a naphthylsulfonyl group and a 2-pyridylsulfonyl group; an amino group such as an amino group, an ethylamino group, a dimethyamino group, a butylamino group, a cyclopentylamino group, a 2-ethylhexylamino group, a dodecylamino group, an anilino group, a naphthylamino group and a 2-pyridylamino group; a halogen atom such as a fluorine atom, a chlorine atom and a bromine atom; a fluorohydrocarbon group such as a fluoromethyl group, a trifluoromethyl group, a pentafluoroethyl group and a pentafluorophenyl group; a cyano group; and a silyl group such as a trimethylsilyl group, triisopropylsilyl group, a triphenylsilyl group and a phenyldiethylsilyl group.

These substituents each may be further substituted by the above substituents and plural of them may form a ring by bonding with each other.

Among them, the alkyl groups are most preferable and the alkyl groups having 2 to 20 carbon atoms are more preferable, and those having 6 to 12 carbon atoms are most preferable.

«Terminal group of thiophene oligomer»

The terminal group of the thiophene oligomer preferably employed in the invention is described below.

The terminal group of the thiophene polymer to be employed in the invention is preferably one having no thienyl group; and examples of preferable terminal group include an aryl group such as a phenyl group, a p-chlorophenyl group, a mesityl group, a tolyl group, a xylyl group, a naphthyl group, an anthoryl group, an azulenyl group, an acenaphthenyl group, a fluorenyl group, a phnanthryl group, an indenyl group, a pyrenyl group and a biphenylyl group; an alkyl group such as a methyl group, an ethyl group, a propyl group, an isopropyl group, a tert-butyl group, a pentyl group, a hexyl group, an octyl group, a dodecyl group, a tetradecyl group and a pentadecyl group; and a halogen atom such as a fluorine atom, a chlorine atom and a bromine atom.

«Steric structural property of repeating unit of thiophene oligomer»

The thiophene oligomer to be used in the invention is preferably one having no Head-to-Head structure, and ones having a Head-to-Tail structure or a Tail-to-Tail structure are preferable.

About the Head-to-Head, Head-to-Tail and Tail-to-Tail Structures relating to the invention, "Π-electron System Organic Solid", edited by the Chemical Society of Japan, Publication Center of the Chemical Society of Japan, p.p. 27 to 32, 1998, and Adv. Mater. 1998, 10, No. 2, P.P. 93 - 116, can be referred. The structural characteristics of them are described below in concrete.

Head-to-Head structure

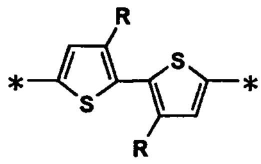

Head-to-Tail structure

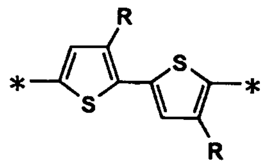

Tail-to-Tail structure

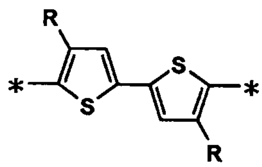

The concrete examples of the thiophene oligomer are listed below, but the invention is not limited to them.

⟨1⟩

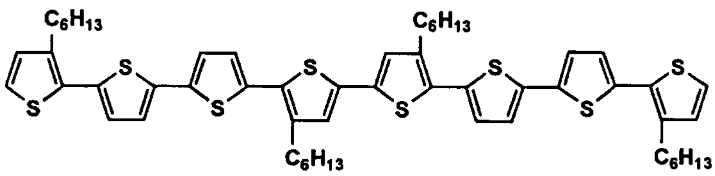

⟨2⟩

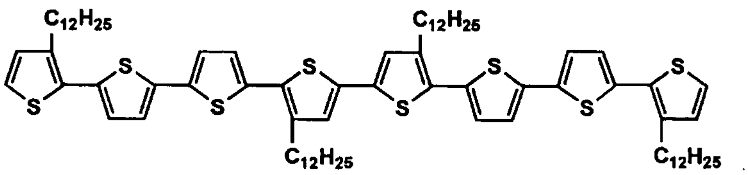

⟨3⟩

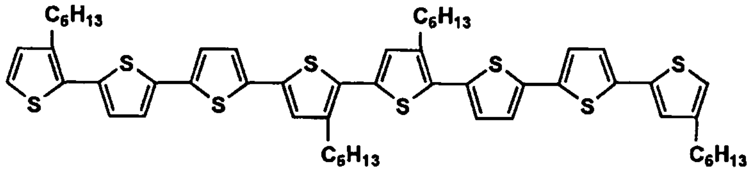

⟨4⟩

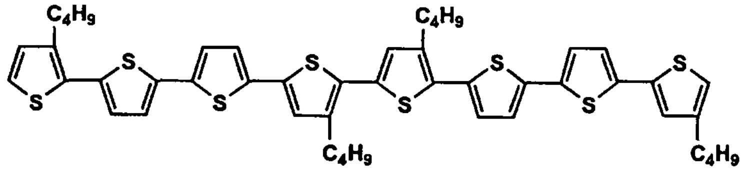

⟨5⟩

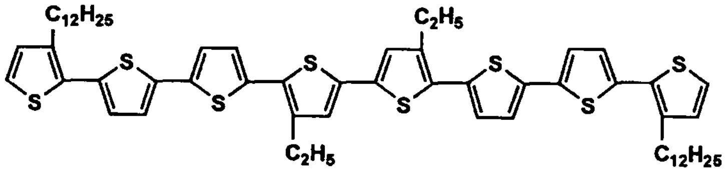

⟨6⟩

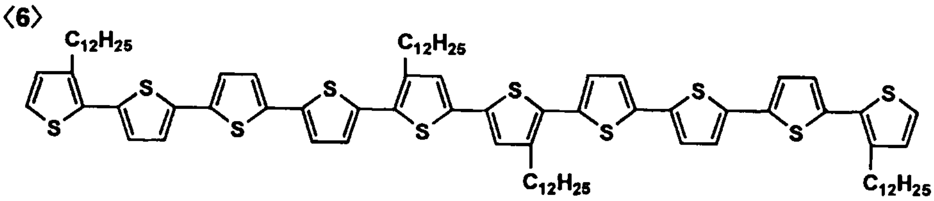

⟨7⟩

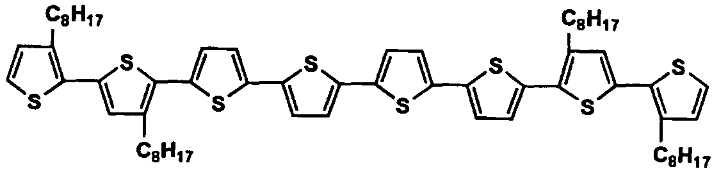

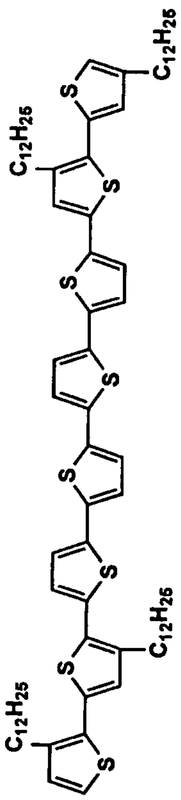

〈8〉

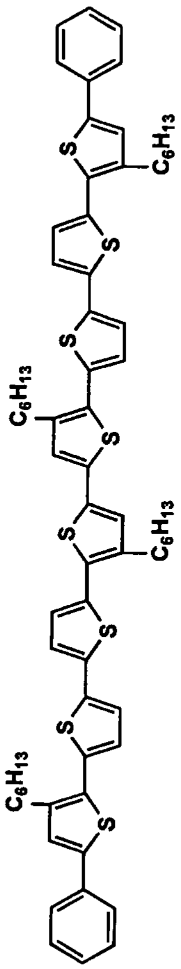

〈9〉

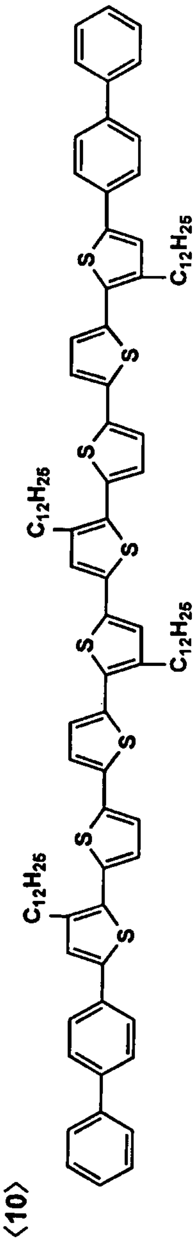

〈10〉

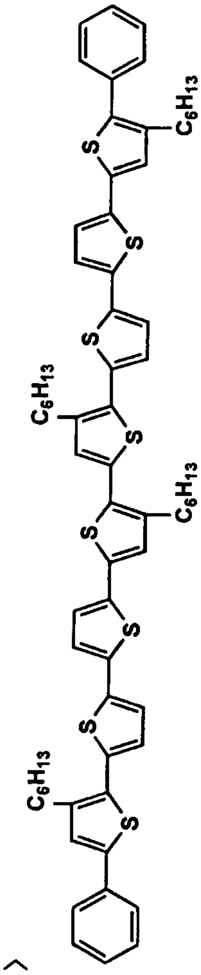

〈11〉

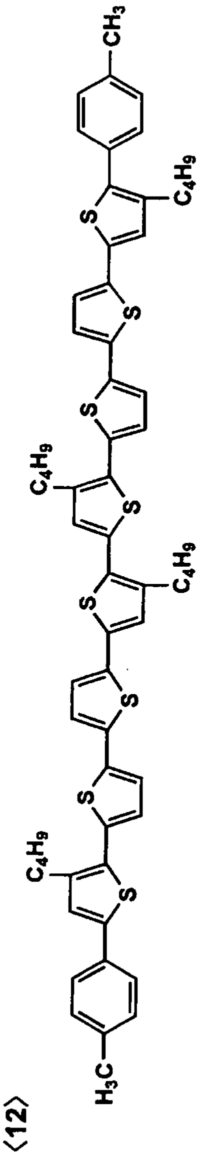

〈12〉

〈13〉

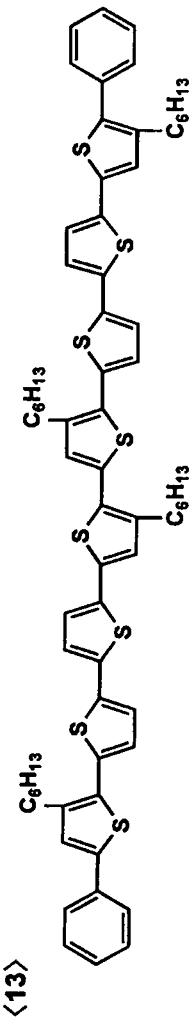

〈14〉

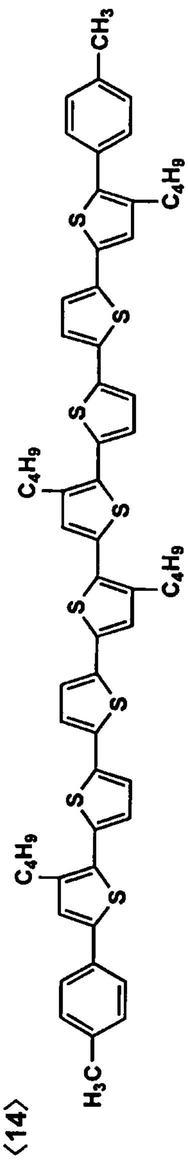

〈15〉

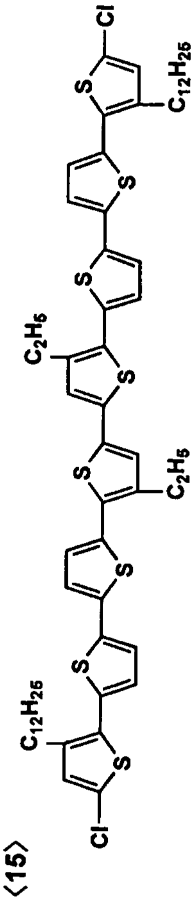

〈16〉

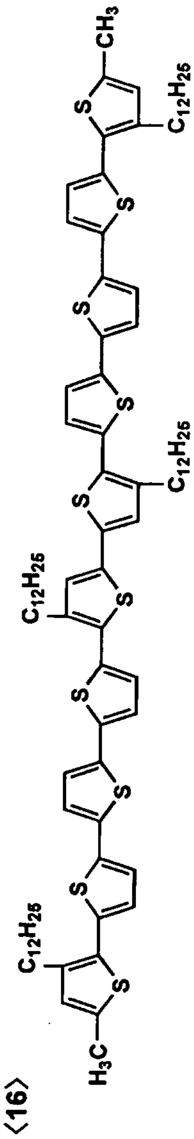

〈17〉

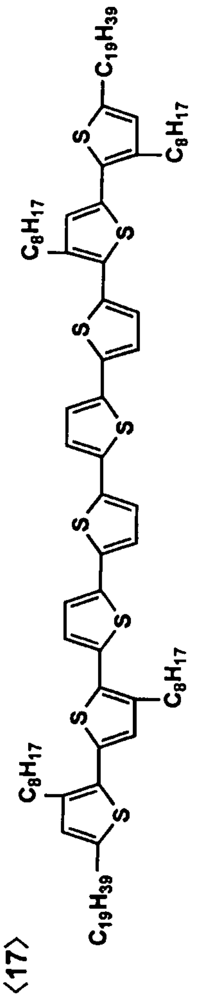

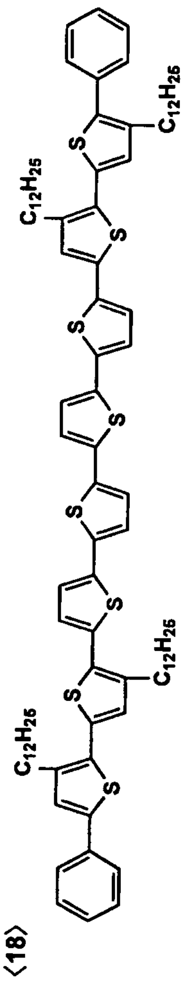

⟨18⟩

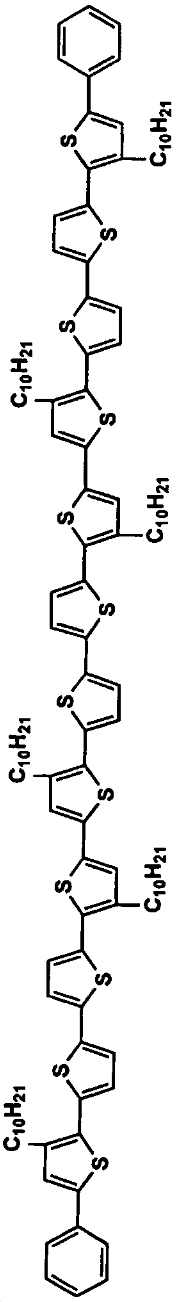

⟨19⟩

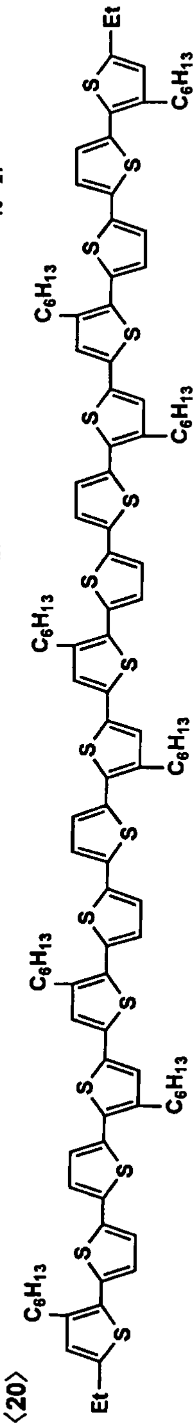

⟨20⟩

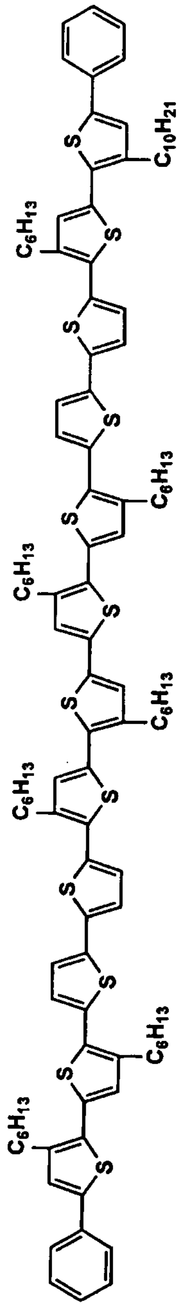

⟨21⟩

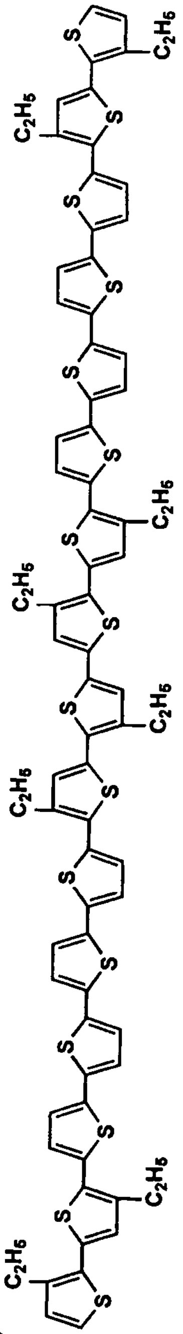

⟨22⟩

<23>

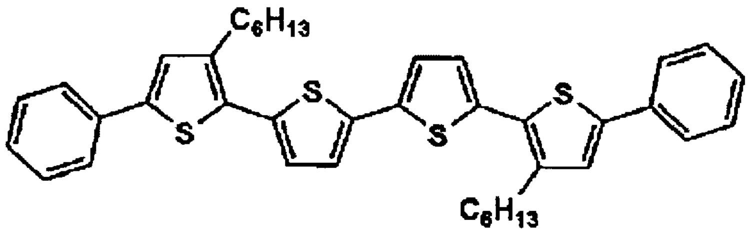

<23>

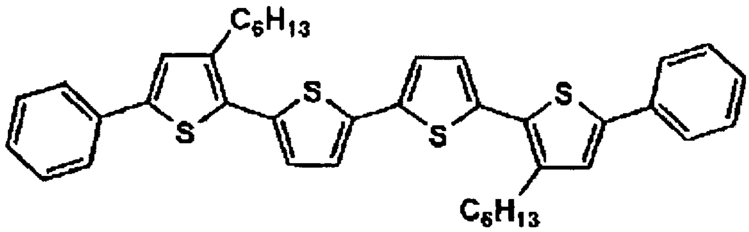

<<Organic thin film transistor (also referred to as organic TFT)>>

The organic thin film transistor (organic TFT) of the invention is described below.

A suitably functioning organic thin film transistor (organic TFT) can be provided by the use of the thiophene oligomer according to the invention. The organic TFT (organic thin film transistor) can be roughly classified into a top-gate type and a bottom-gate type. The top-gate type has a source electrode and a drain electrode which are connected with together by an organic semiconductor channel of a semiconductor layer on a substrate and a gate electrode is provided over them through a gate isolating layer. The bottom-gate type has a gate electrode on a substrate and a source electrode and a drain electrode which are connected by an organic semiconductor channel are provided thereon through a gate isolating layer.

For providing the thiophene oligomer as the semiconductor layer of the organic TFT, it is preferable that a solution which is prepared by dissolving the thiophene oligomer in a suitable solvent and adding an additive according to necessity is provided on the substrate by a method such as a cast coating method, a spin coating method, a printing method, an ink-jetting method and an ablation method, even though the provision can be performed by a vacuum deposition.

In such the case, the solvent for dissolving the organic semiconductor relating to the invention is not specifically limited as long as the solvent can dissolve the organic semiconductor for obtaining a solution having a suitable concentration. Concrete examples of the solvent include a chain-formed ether solvent such as diethyl ether and di-iso-propyl ether; a cyclic ether solvent such as tetrahydrofuran and dioxane; a ketone solvent such as acetone and methyl ethyl ketone; a halogenized alkyl type solvent such as chloroform and 1,2-dichloroethane, an aromatic solvent such as toluene, o-dichlorobenzene, nitrobenzene and m-cresol, N-methylpyrrolidone and carbon disulfide.

In the invention, the material for constituting the source electrode, drain electrode and gate electrode is not specifically limited as long as the material is electroconductive. Examples of usable material include platinum, gold, silver, nickel, chromium, copper, iron. tin, antimony, lead, tantalum, indium, palladium, tellurium, rhenium, iridium, aluminum, ruthenium, germanium, molybdenum, tungsten, tin·antimony oxide, indium·tin oxide (ITO), fluorine-doped zinc oxide, zinc, carbon, graphite, glassy carbon, silver past, carbon paste, lithium, beryllium, sodium, magnesium, potassium, calcium, scandium, titanium, manganese, zirconium, gallium, niobium, a sodium-potassium alloy, a magnesium/copper mixture, a magnesium/silver mixture, a magnesium/aluminum mixture, a magnesium/indium mixture, an aluminum/aluminum oxide mixture and a lithium/aluminum mixture. Platinum, gold, silver, copper, aluminum, indium, ITO and carbon are preferable. A known electroconductive polymer increased in the electroconductivity by doping such as electroconductive polyaniline, electroconductive polypyrrol and electroconductive polythiophnen, and a complex of polyethylenedioxythiophene and polyethylene sulfonic acid are also preferably usable. Among them, one displaying low electric resistance at the contacting surface with the semiconductor layer is preferred.

The following methods are applicable for forming the electrodes; a method in which a electroconductive thin layer prepared by evaporation depositing or spattering the foregoing material is formed into the electrode by a known photolithographic method or a lift-off method, and a method in which a resist is provided on a foil of metal such as aluminum and copper by a thermal transfer or ink-jet and the metal foil is subjected to etching. Moreover, the electrodes may be formed by directly patterning the solution or the dispersion of fine particles of the electroconductive polymer by an ink-jet method, or by lithographing or laser ablation the coated layer. Furthermore, a method can be applied, in which electroconductive polymer, an ink containing electroconductive fine particles or an electroconductive paste is patterned by a printing method such as relief printing, intaglio printing, lithographic printing and screen printing.

For the gate isolating layer an inorganic oxide layer having high specific permittivity is preferred even though various kinds of layers can be employed. As the inorganic oxide, for example, silicon oxide, aluminum oxide, tantalum oxide, titanium oxide, tin oxide, vanadium oxide, barium strontium titanate, barium zirconate titanate, lead zirconium titanate, lead lanthanum titanate, strontium titanate, barium titanate, barium magnesium fluoride, bismuth titanate, strontium bismuth titanate, strontium bismuth tantalite and yttrium trioxide are employable. Among the above, silicon oxide, aluminum oxide, tantalum oxide and titanium oxide are preferable. An inorganic nitride such as silicon nitride and aluminum nitride is also preferably usable.

For forming the layer, a dry process such as a vacuum deposition method, a molecular ray epitaxial growing method, an ion cluster beam method, a low energy ion beam method, an ion plating method, a CVD method, a spattering method and an atmospheric pressure plasma method, and a wet process such as a spray coating method, a spin coating method, a blade coating method, a dip coating method, a casting method, a roller coating method, a bar coating method, a die coating method and a patterning by printing or ink-jetting are applicable. These methods can be selected corresponding to the materials.

As the wet process, a method in which a liquid prepared by dispersing fine particles of the inorganic oxide into an optional organic solvent or water employing an dispersing agent, according to necessity, is coated and dried, and a method so called as sol-gel method in which a solution of a precursor such as an alkoxide compound is coated and dried are applicable. Among the above methods, the atmospheric pressure plasma method and the sol-gel method is preferred.

The isolating layer forming method by a plasma layer forming treatment in the atmospheric pressure is a treatment by excited plasma of a reactive gas generated by discharging in atmospheric pressure or near atmospheric pressure for forming a layer on a substrate; hereinafter this method is also referred to as an atmospheric pressure plasma method. Such the method is described in Japanese Patent Tokkai Hei 11-61406 and 11-133205, Tokkai 2000-12804, 2000-147209 and 2000-185362. By this method, a thin layer superior in the property can be produced with high producing efficiency.

For the organic compound layer the following s are employable; polyimide, polyamide, polyester, polyacrylate, a light radical polymerization type and light-cation polymerization type light-hardenable resins, a copolymer containing acrylonitrile component, polyvinylphenol, poly(vinyl alcohol), novolac resin and cyanoethylpullulan. For forming the organic layer, the foregoing wet method is preferable. The inorganic compound layer and the organic compound layer can be employed in combination in a laminated form. The thickness of the layer is usually from 50 nm to 3 $\mu$m, and preferably from 100 nm to 1 $\mu$m.

The substrate is constituted by glass or flexible resin sheet, and a plastic film can be employed as the sheet. Examples of the plastic film include a film of poly(ethylene terephthalate) (PET), poly(ethylene naphthalate) (PEN), polyethersulfon (PES), polyetherimide, poly(ether ether ketone), poly(phenylene sulfide), polyallylate, polyimide, polycarbonate (PC), cellulose triacetate (TAC) and cellulose acetate propionate (CAP). By the use of the plastic film, the weight can be made lighter and the portableness and the shock resistivity can be improved compared to the product using the glass substrate.

The organic TFT (including an electric field effect transistor) employing the organic thin layer formed by the organic TFT material of the invention is described below.

Fig. 1 shows an example of the constitution of the organic TFT according to the invention. Fig. 1(a) shows an electric field effect transistor in which a source electrode 2 and a drain electrode 3 are formed on a substrate 6 and an organic semiconductor layer 1 of the organic thin film transistor material according to the invention is provided between the electrodes, and an isolation layer 5 is formed thereon, and a gate electrode 4 is formed on the isolation layer 5 to form the electric field effect transistor. Fig. 1(b) shows one in which the organic semiconductor layer is formed by a coating method so that the layer entirely covers the surface of the electrodes and the substrate; such the layer is formed only between the two electrodes in Fig. 1(a). In Fig. 1(d), the organic semiconductor layer 1 is firstly formed by coating on the substrate 6 and then the source electrode 2, drain electrode 3, the isolation layer 5 and gate electrode 4 are provided.

In Fig. 1(d), the gate electrode 4 of metal foil is formed on the support 6 that then the isolation layer 5 is formed

thereon, the source electrode 2 and the drain electrode 3 each formed by the metal foil on the isolation layer, and then the semiconductor layer 1 is formed between the electrodes by the organic thin film transistor material of the invention. Furthermore, the structures shown in Fig. 1(e) and (f) can be taken.

Fig. 2 shows an example of schematic equivalent circuit drawing.

The organic TFT sheet 10 includes many organic TFT 11 arranged in a matrix. 7 is the gate busline of each of the TFT 11, and 8 is the source busline of each of the TFT 11. To the source electrode of each of the TFT 11, an output element 12 is connected which is, for example, a liquid crystal or an electrophoretic element constituting the pixel of the displaying apparatus. The pixel electrode may be used as an input electrode of a photo sensor. In the displayed example, the liquid crystal as the output element is shown by an equivalent circuit composed of a resistor and a condenser. 13 is an accumulation condenser, 14 is vertical driving circuit and 15 is a parallel driving circuit.

<<Organic EL Device (Organic Electroluminescence Device)>>

While the organic EL device according to the present invention can be, for example, a device in which an organic EL layer (also called an organic compound layer) is held between an anode and a cathode, the structure of these can be prepared using the conventionally well-known layer structure, and material of organic EL layer. See, for example, the reference in Nature, No. 395, p. 151 to 154, etc.

In making the organic EL device of the present invention emit light, from the point of view of obtaining high emitted light luminosity, and also, obtaining the effects of long light emitting life, etc., it is desirable that the organic semiconductor device or the organic thin film transistor of the present invention is provided.

**[Examples]**

Although the present invention is explained below using some implementation examples, the present invention shall not be construed to be limited to or by the following implementation examples.

Here, the structural equation of the organic semiconductor compound used in the implementation examples is given below.

Chemical Equation 11

Comparison compound <1>

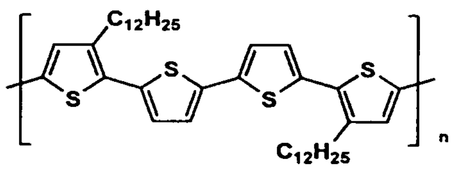

Example 1

<<Preparation of Organic Thin Film Transistor 1>>: Present Invention

On a Si wafer with a specific resistivity of 0.02 Ω.cm as the gate electrode, after forming a gate insulation layer by forming a thermal oxide film with a thickness of 200 nm, surface treatment is made using octadecyltrichlorosilane.

Next, as the organic semiconductor, a cyclohexane solution of the compound <2> (content of 98.6%, Mw/Mn = 1) was bubbled with nitrogen gas thereby removing any dissolved oxygen in the solution, the coating was made on the surface of said thermal oxide film (silicon oxide film) using an applicator in a nitrogen gas environment with a pressure of 1.013 x $10^2$ kPa, and the film was dried at room temperature. At this time, the thickness of the semiconductor layer was 20 nm.

As a result of evaluating the obtained film by X-ray diffraction, the X-ray diffraction spectrum chart shown in Figure 3 was obtained. The half width at the diffraction peak with the maximum intensity at 25.1 Å was 0.22°.

In addition, gold was plated on the top surface of this film using a mask, thereby forming the source and drain

electrodes. With the above, an organic thin film transistor (present invention) was prepared with a channel length of L = 30 μm and a channel width of W = 1 mm.

The organic thin film transistor 1 operated satisfactorily as a p-channel enhancement type TFT. When the carrier mobility was derived from the organic thin film transistor so obtained from the saturation region of the I-V characteristics, its value was found to be 0.10 $cm^2/V.s$.

<<Preparation of Organic Thin Film Transistor 2>>: Present Invention

In the preparation of the organic thin film transistor 1, the compound <2> is changed to the compound <9> (content of 99.9%, Mw/Mn = 1), it is dissolved in an organic solvent which was a mixture of THF and cyclohexane (2 : 8), bubbled with nitrogen gas thereby removing any dissolved oxygen in the solution, and the coating was made on the surface of said thermal oxide film (silicon oxide film) using an applicator in a nitrogen gas environment with a pressure of 1.013 x $10^2$ kPa. After the film was dried at room temperature, it was heat-treated for 30 minutes at a temperature of 93 °C in a $N_2$ gas atmosphere. At this time, the thickness of the semiconductor layer was 20 nm.

As a result of evaluating the obtained film by X-ray diffraction, the X-ray diffraction spectrum chart shown in Figure 4 was obtained. The half width at the diffraction peak with the maximum intensity at 16.5 Å was 0.12°.

In addition, similar to the preparation of the organic thin film transistor 1 above, gold was plated on the top surface of this film using a mask, thereby forming the source and drain electrodes. With the above, an organic thin film transistor (present invention) was prepared with a channel length of L = 30 μm and a channel width of W = 1 mm. The prepared organic thin film transistor 2 operated satisfactorily as a p-channel enhancement type TFT, and the carrier mobility derived from the saturation region of the I-V characteristics was 0.15 $cm^2/V.s$.

«Preparation of Organic Thin Film Transistor 3»

In the preparation of the organic thin film transistor 1, the compound <2> is changed to the compound <23> (content of 99.9%, Mw/Mn = 1), it is dissolved in an organic solvent which was a mixture of THF and cyclohexane (1 : 9), bubbled with nitrogen gas thereby removing any dissolved oxygen in the solution, and the coating was made on the surface of said thermal oxide film (silicon oxide film) using an applicator in a nitrogen gas environment with a pressure of 1.013 x $10^2$ kPa. After the film was dried at room temperature, it was heat-treated for 30 minutes at a temperature of 48 °C in a $N_2$ gas atmosphere. At this time, the thickness of the semiconductor layer was 20 nm.

As a result of evaluating the obtained film by X-ray diffraction, the X-ray diffraction spectrum chart shown in Figure 5 was obtained. The half width at the diffraction peak with the maximum intensity at 9.7 Å was 0.11°.

In addition, similar to the preparation of the organic thin film transistor 1 above, gold was plated on the top surface of this film using a mask, thereby forming the source and drain electrodes. With the above, an organic thin film transistor (present invention) was prepared with a channel length of L = 30 μm and a channel width of W = 1 mm. The prepared transistor operated satisfactorily as a p-channel enhancement type TFT, and the carrier mobility derived from the saturation region of the I-V characteristics was 0.16 $cm^2/V.s$.

«Preparation of Organic Thin Film Transistor 4»

On a Si wafer with a specific resistivity of 0.02 Ω.cm as the gate electrode, a gate insulation layer was formed by a thermal oxide film with a thickness of 200 nm. Next, a compound <31> (content of 99.8%, Mw/Mn = 1) was used as the organic semiconductor, dissolved in an organic solvent which was a mixture of THF and cyclohexane (2 : 8), was bubbled with nitrogen gas thereby removing any dissolved oxygen in the solution, the coating was made on the surface of said thermal oxide film (silicon oxide film) using an applicator in a nitrogen gas environment with a pressure of 1.013 x $10^2$ kPa, and the film was dried at room temperature. At this time, the thickness of the semiconductor layer was 20 nm.

As a result of evaluating the obtained film by X-ray diffraction, the half width at the diffraction peak with the maximum intensity at 18.2 Å was 0.4°.

In addition, similar to the preparation of the organic thin film transistor 1 above, gold was plated on the top surface of this film using a mask, thereby forming the source and drain electrodes. With the above, an organic thin film transistor (present invention) was prepared with a channel length of L = 30 μm and a channel width of W = 1 mm. The prepared transistor operated satisfactorily as a p-channel enhancement type TFT, and the carrier mobility derived from the saturation region of the I-V characteristics was 0.05 $cm^2/V.s$.

«Preparation of Organic Thin Film Transistor 5 for Comparison»

In the preparation of the organic thin film transistor 1, the compound <2> is changed to conventionally well-known comparison compound 1 (Thiophene polymer described in J. Am. Chem, Soc. 2004, 126, 3378-3379: Mw 18000, Mn 10400, Mw/Mn = 1.7), chloroform was used as the organic solvent, a chloroform solution was prepared, was bubbled with nitrogen gas thereby removing any dissolved oxygen in the solution, the coating was made on the surface of said gate insulation film using an applicator in a nitrogen gas environment, and the film was dried at room temperature. At this time, the thickness of the semiconductor layer was 20 nm.

As a result of evaluating the obtained film by X-ray diffraction, the X-ray diffraction spectrum chart shown in Figure 6 was obtained. The half width at the diffraction peak with the maximum intensity at 19.6 Å was 0.69°.

In addition, similar to the preparation of the organic thin film transistor 1 above, gold was plated on the top surface of this film using a mask, thereby forming the source and drain electrodes. With the above, an organic thin film transistor 1 was prepared with a channel length of L = 30 μm and a channel width of W = 1 mm. This transistor operated satisfactorily as a p-channel enhancement type TFT, and the carrier mobility derived from the saturation region of the I-V characteristics was 0.02 $cm^2/V.s$.

From the above result, compared with the organic TFT device for comparison, it is evident that the organic TFT device of the present invention shows superior transistor characteristics immediately after preparation, and also exhibits superior transistor characteristics of the carrier mobility being high.

Example 2

«Preparation of Organic EL Device»

The method described in Nature, No. 395, pp. 151-154 was referred to for preparing the organic EL device, and a top emission type organic EL device was prepared with a sealed structure as is shown in Figure 7. Further, in Figure 7, 101 is the substrate, 102a is the anode, 102b is the organic EL layer (in specific terms, this includes the electron transport layer, the light emitting layer, the hole transport layer), 102c is the cathode, and the light emitting device 102 is formed by the anode 102a, the organic EL layer 102b, and the cathode 102c. Also, the sealed film is indicated by 103. Further, the organic EL device of the present invention can be of the bottom emission type or of the top emission type.

Although an organic EL and an organic thin film transistor of the present invention were combined (here, the organic thin film transistor of the present invention is used as a switching transistor or as a drive transistor), thereby preparing a light emitting device of the active matrix type, in this case, for example, as is shown in Figure 8, the form of using a substrate is shown here as an example in which the TFT 602 (can also be an organic thin film transistor 602) on the glass substrate 601. Here, the method of manufacturing the TFT 602 can be determined by referring to the method of manufacture of a widely known TFT. Of course, as a TFT, it can be a conventionally well-known top gate type TFT or a bottom gate type TFT.

The organic EL device prepared in the above exhibited excellent light emitted characteristics in various light emitting modes of single color, full color, white color, etc.

## Claims

1. In an organic semiconductor thin film containing an organic semiconductor compound, the organic semiconductor thin film **characterized in that** the organic semiconductor thin film is manufactured by a process of forming a film by using a solution or a dispersion at room temperature prepared by mixing the organic semiconductor compound and an organic solvent, and the half width of a diffraction peak having the maximum intensity is 0.4° or less in an X-ray diffraction spectrum of the film.

2. In an organic semiconductor thin film, containing an organic semiconductor compound, the organic semiconductor thin film **characterized in that** the organic semiconductor thin film is manufactured by a process of forming a film by using a solution or a dispersion at room temperature prepared by mixing the organic semiconductor compound and an organic solvent, and the half width of a diffraction peak having the maximum intensity is 0.2° or less in an X-ray diffraction spectrum of the film.

3. The organic semiconductor thin film described in claim 1 or 2, **characterized in that** the organic solvent contains a non-halogen type solvent.

**4.** The organic semiconductor thin film described in any one of claims 1 to 3, **characterized in that** the organic semiconductor compound has a weight average molecular weight Mw of 10000 or less.

**5.** The organic semiconductor thin film described in any one of claims 1 to 4, **characterized in that** the organic semiconductor compound has a ratio (Mw/Mn) of 2 or less, where Mw represents a weight average molecular weight and Mn represents a number average molecular weight.

**6.** The organic semiconductor thin film described in any one of claims 1 to 5, **characterized in that** the content of the organic semiconductor compound is 95% or more.

**7.** The organic semiconductor thin film described in any one of claims 1 to 6, **characterized in that** the organic semiconductor compound is a $\pi$-conjugate compound having two or more aromatic rings.

**8.** The organic semiconductor thin film described in claim 7, **characterized in that** the organic semiconductor compound has two or more kinds of aromatic hydrocarbon rings or two or more kinds of aromatic heterocyclic rings as a partial structure.

**9.** The organic semiconductor thin film described in claim 7, **characterized in that** the organic semiconductor compound has three or more kinds of aromatic hydrocarbon rings or three or more kinds of aromatic heterocyclic rings as a partial structure.

**10.** The organic semiconductor thin film described in any one of claims 7 to 9, **characterized in that** the organic semiconductor compound has an unsubstituted aromatic hydrocarbon ring having no condensed ring or an unsubstituted aromatic heterocyclic ring as a partial structure.

**11.** The organic semiconductor thin film described in any one of claims 1 to 10, **characterized in that** the organic semiconductor compound includes a thiophene oligomer, and the thiophene oligomer has a thiophene ring having a substituent and a partial structure in which a repeating unit of an unsubstituted thiophene ring continues at least two or more.

**12.** The organic semiconductor thin film described in claim 11, **characterized in that** the number of the thiophene rings contained in the thiophene oligomer is 3 to 20.

**13.** The organic semiconductor thin film described in claim 11, **characterized in that** the number of the thiophene rings contained in the thiophene oligomer is 4 to 10.

**14.** The organic semiconductor thin film described in any one of claims 11 to 13, **characterized in that** the thiophene oligomer has a partial structure represented by the following general formula (1):

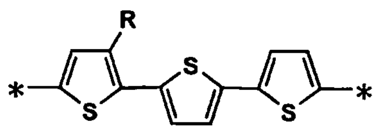

where R in the formula represents a substituent.

**15.** The organic semiconductor thin film described in any one of claims 11 to 14, **characterized in that** a terminal group of the thiophene oligomer has not a thienyl group.

**16.** The organic semiconductor thin film described in any one of claims 11 to 15, **characterized in that** the thiophene oligomer has not Head-to-Head structure.

**17.** An organic semiconductor device, comprising:

the organic semiconductor thin film described in any one of claims 1 to 16.

**18.** An organic thin film transistor, comprising:

an organic semiconductor layer of the organic semiconductor thin film described in any one of claims 11 to 16.

**19.** An organic electronic luminescence element, comprising:

the organic semiconductor device described in claim 17 or the organic thin film transistor described in claim 18.

FIG. 1(a)

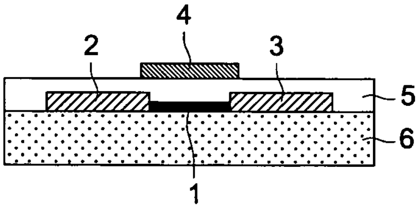


FIG. 1(d)

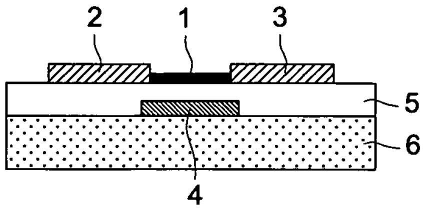


FIG. 1(b)

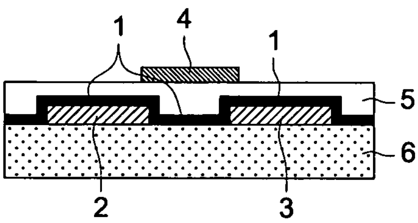


FIG. 1(e)

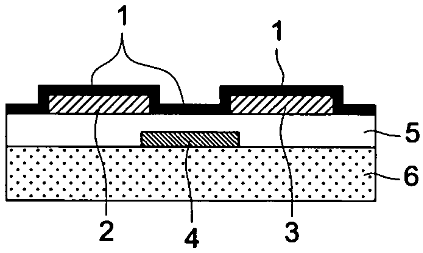


FIG. 1(c)

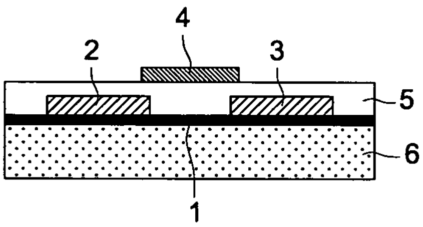


FIG. 1(f)

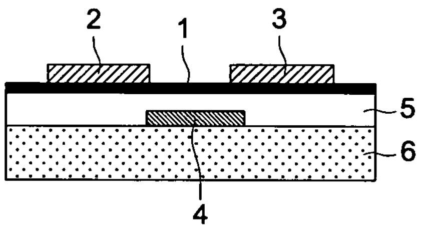


FIG. 2

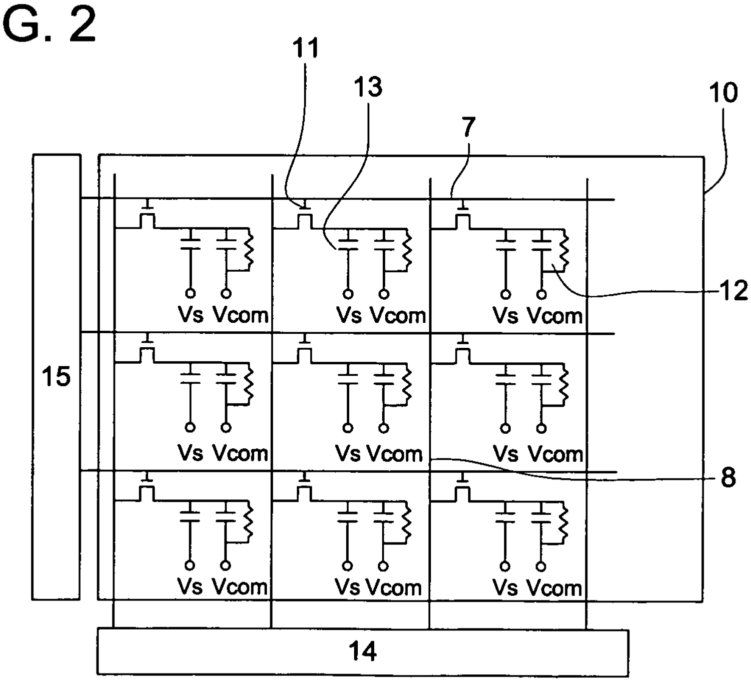

FIG. 3

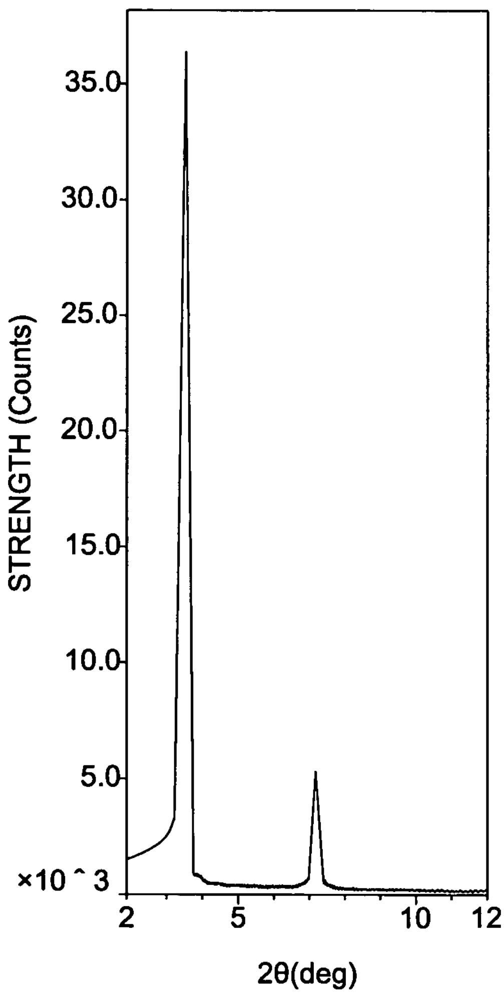

FIG. 4

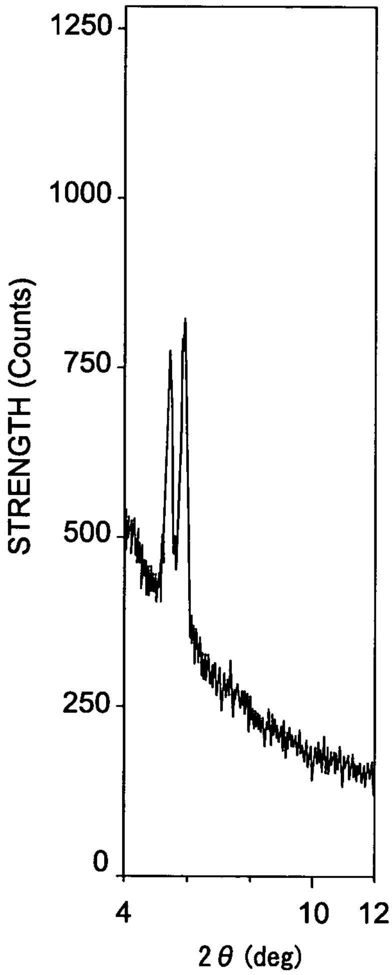

FIG. 5

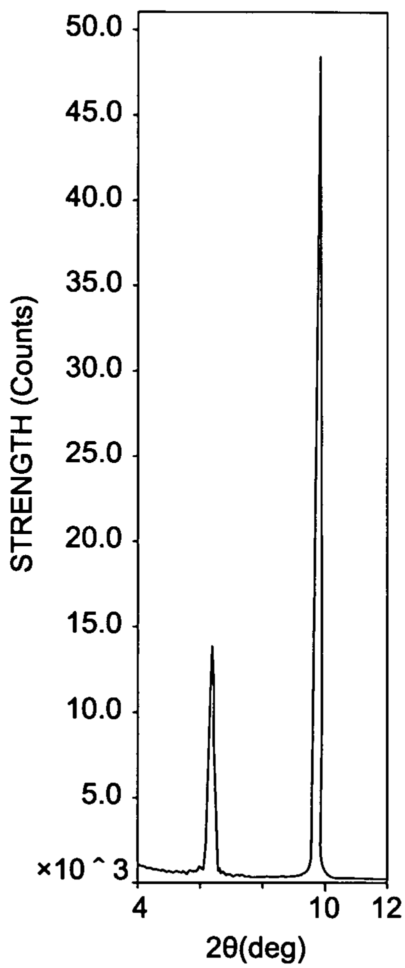
50.0
45.0
40.0
35.0
30.0
25.0
20.0
15.0
10.0
5.0
×10 ^ 3
STRENGTH (Counts)
4
10
12
2θ(deg)

FIG. 6

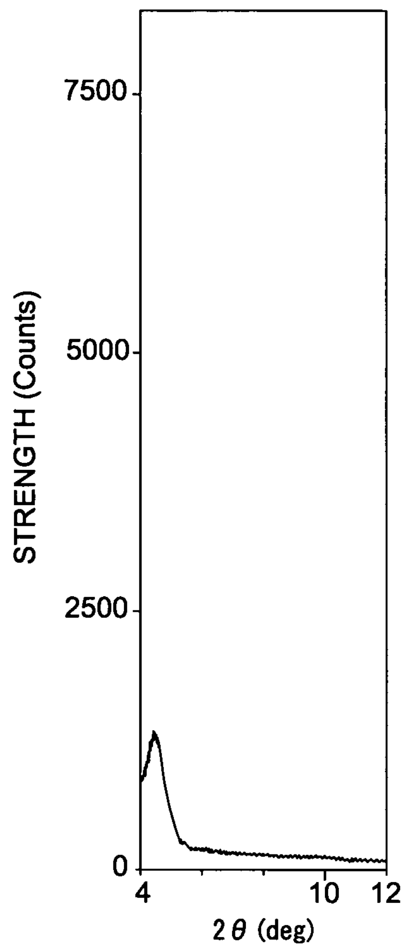
7500
5000
2500
0
STRENGTH (Counts)
4
10
12
2θ (deg)

FIG. 7

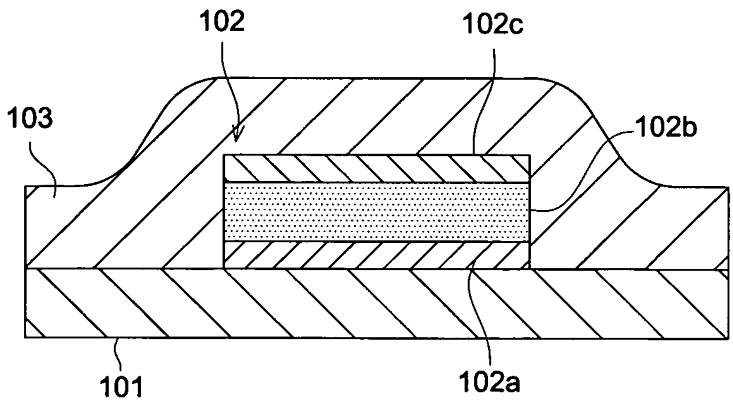
102
102c
103
102b
101
102a

FIG. 8

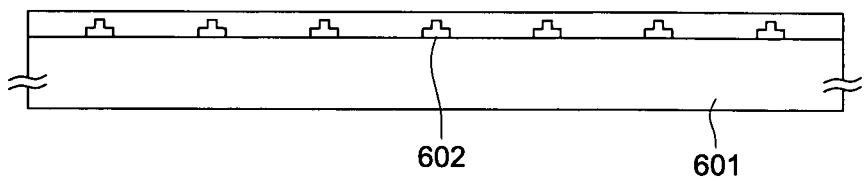

## INTERNATIONAL SEARCH REPORT

International application No.
PCT/JP2005/010324

A. CLASSIFICATION OF SUBJECT MATTER
Int.Cl[7] H01L29/786, 51/00//C07D333/10

According to International Patent Classification (IPC) or to both national classification and IPC

B. FIELDS SEARCHED

Minimum documentation searched (classification system followed by classification symbols)
Int.Cl[7] H01L29/786, 51/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2003-261655 A (Xerox Corp.), 19 September, 2003 (19.09.03), & US 2003/160230 A1 & EP 1329475 A1 | 1-19 |
| Y | JP 2003-268083 A (Xerox Corp.), 25 September, 2003 (25.09.03), & US 2003/166829 A1 & EP 1327646 A1 | 1-19 |
| Y | JP 2002-100782 A (International Business Machines Corp.), 05 April, 2002 (05.04.02), & US 2002/72618 A1 | 1-19 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 August, 2005 (29.08.05) | 13 September, 2005 (13.09.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/010324

Claims 1-6, 17-19 include an organic semiconductor thin film formed by using a plenty of solutions or dispersion liquids defined by the half-value width, molecular mass, and content of desired values. Claims 1-6, 17-19 include an organic semiconductor film formed by using all the solutions or dispersion liquids having such a characteristic. However, what is disclosed in the Description within the meaning of PCT Article 5 is only a small part of the organic semiconductor film claimed and the claims are not supported by the Description within the meaning of PCT Article 6.

Claims 7-16 include an organic semiconductor thin film of the aforementioned characteristic containing a plenty of organic semiconductor compounds. However, what is disclosed in the Description within the meaning of PCT Article 5 is only a small part of the organic semiconductor thin film claimed and the claims are not supported by the Description within the meaning of PCT Article 6.

Consequently, the search has been performed on the part disclosed in and supported by the Description, i.e., the organic semiconductor thin films containing compounds <2>, <9>, <23>, and <31>.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5055568 A
- JP 8264805 A
- JP 11061406 A
- JP 11133205 A
- JP 2000012804 A
- JP 2000147209 A
- JP 2000185362 A


### Non-patent literature cited in the description


- *Science,* 599
- *Nature,* 521
- *Advanced Material magazine,* 2002, 99
- *Appl. Phys. Lett.,* 1998, 1854
- *Proc. ICSM,* 2004
- *Chemical Material magazine,* 1998, 633
- Π-electron System Organic Solid. Publication Center of the Chemical Society of Japan, 1998, 27-32
- *Adv. Mater,* 1998, vol. 10 (2), 93-116
- *Nature,* 151-154
- *J. Am. Chem, Soc.,* 2004, vol. 126, 3378-3379
- *Nature,* 151-154